# EUROPÄISCHE PATENTANMELDUNG

(11) **EP 1 262 158 A2**
(43) Veröffentlichungstag der Anmeldung: **04.12.2002**
(21) Anmeldenummer: 02010760.3
(22) Anmeldetag: 14.05.2002
(51) Int. Cl.: A61H 1/00, A63B 26/00, G09B 7/00, G06F 3/00, G06F 17/00

(54) **Vorrichtung zur interaktiven Rehabilitationsunterstützung mit Gestikerkennung**

(30) Priorität: 25.05.2001 DE 10125653
(71) Anmelder: SIEMENS AKTIENGESELLSCHAFT, 80333 München (DE)
(72) Erfinder: Breimesser, Fritz, 90491 Nuernberg (DE); Eisermann, Uwe, 91052 Erlangen (DE); Roettger, Hans, 80802 Muenchen (DE); Schmidt, Kai-Uwe, Dr., Paoli, PA 19301 (US)

(57) **Zusammenfassung**

Vorrichtung zur interaktiven Rehabilitationsunterstützung durch computergestütztes, selbständig vom Patienten durchgeführtes Training mit einer Eingabevorrichtung für die Eingabebefehle des Patienten, wobei die Eingabevorrichtung ein Gestikerkennungssystem als zusätzliches Eingabemedium umfasst.

## Beschreibung

Vorrichtung zur interaktiven Rehabilitationsunterstützung mit Gestikerkennung

Die Erfindung bezieht sich auf eine Vorrichtung zur interaktiven Rehabilitationsunterstützung durch computergestütztes, selbständig vom Patienten durchgeführtes Training mit einer Eingabevorrichtung für die Eingabebefehle des Patienten.

Im Rahmen eines Rehabilitationsprozesses müssen sowohl kognitive als auch motorische Störungen therapiert werden. Dabei kann computergestütztes Training, das selbständig vom Patienten durchgeführt und vom Therapeuten betreut wird, wesentlich zum Erfolg beitragen.

Bei der Rehabilitation kognitiver Störungen hat sich das computerunterstützte Training bereits etabliert. Neuere Entwicklungen ermöglichen zudem das interaktive, individuelle Training bis hin zur sogenannten Telerehabilitation, wo der Patient zu Hause die ihm vorgeschriebenen Übungen durchführt. Ein dafür geeignetes System ist beispielsweise in der US-Patentschrift 5,711,671 beschrieben. Ein ähnliches System ("Rehab Assistant") ist von der Anmelderin konzipiert und bereits mit Kliniken erfolgreich erprobt worden. Bislang können jedoch Personen mit extremer Behinderung oder motorischen Störungen mangels entsprechender Eingabemedien diese Trainingsmethode nicht durchführen.

Bisher wurden beim kognitiven Training die bei PCs üblichen Eingabemedien wie die Tastatur, eventuell auch in Spezialausführungen, Maus, Touchscreen oder Joystick benutzt. In den angesprochenen Sonderfällen extremer Behinderung oder motorischer Störungen kommen auch spezielle Sprachsteuerungen oder Trackingsysteme zum Einsatz. Für motorisches Training gibt es aber keine adäquaten Eingabemedien.

Der Erfindung liegt daher die Aufgabe zugrunde eine Vorrichtung der eingangs genannten Art so auszugestalten, dass auch für Benutzer mit komplexen Behinderungen ein neuer individuell anpassbarer Eingabekanal zur Verfügung steht.

Zur Lösung dieser Aufgabe ist erfindungsgemäß vorgesehen, dass die Eingabevorrichtung ein Gestikerkennungssystem als zusätzliches Eingabemedium umfasst, wobei das Gestikerkennungssystem bevorzugt in wiederholten Adaptionsphasen patientenspezifisch programmierbar ist.

Gestikerkennungssysteme sind zwar bereits in vielen Varianten vorgeschlagen worden, wobei es dort aber immer darum geht, dass sich der Benutzer an vorgegebene Standardsituationen anpassen soll, wie beispielsweise bei Gestikerkennungssystemen zur Erkennung und Übersetzung der Zeichensprache oder der Lippensprache von Taubstummen. Patienten, die weder ein Eingabemedium wie eine Maus oder eine Tastatur von Hand betätigen können, noch in der Lage sind, bestimmte Gesten exakt nachzubilden, können mit diesen bekannten Gestikerkennungssystemen nichts anfangen.

Aus diesem Grund ist im Unterschied zu bisher bekannten Gestikerkennungssystemen erfindungsgemäß vorgesehen, dass das System zunächst patientenspezifische Adaptionsphasen umfasst, in denen das System die individuellen Reaktionen und ihre Zuordnung zu bestimmten Eingabebefehlen lernt. Der Befehlsvorrat wird dabei beispielsweise auf wenige Befehle wie Ja, Nein, Links, Rechts, Halt oder dergleichen beschränkt sein, wobei das Erlernen dieser Befehle in der Adaptionsphase, das selbstverständlich in Verbindung mit dem Therapeuten vor dem eigentlichen häuslichen Training stattfinden muss, in der Weise vonstatten geht, dass der Patient auf einen ihm vorgelegten Befehl eine entsprechende körperliche Reaktion beispielsweise ein Winken mit der Hand, ein Kopfnicken oder dergleichen durchführt, das dann in entsprechenden Wiederholungen als Gestikbefehl für das entsprechende Kommando vom System "erlernt" wird.

Dabei hat es sich auch als zweckmäßig erwiesen, dass das Gestikerkennungssystem vor jedem späteren Einsatz als Eingabevorrichtung für das Trainingsprogramm immer erst alle gespeicherten Symbole abspielt und die Gestik des Benutzers mit den gespeicherten Gestikdaten vergleicht, um - gegebenenfalls nach einem erneuten Update - festzustellen, ob der Patient für die einzelnen Befehle nach wie vor die gleichen erkennbaren Gesten ausführt oder ob es notwendig ist, entweder die abgespeicherten Gesten für den entsprechenden Befehl abzuwandeln oder aber Unterstützung beispielsweise durch eine Onlineverbindung mit der Klinik oder dem Therapeuten anzufordern.

Das Gestenerkennungssystem kann dabei in Ausgestaltung der Erfindung auf Grundlage von Hidden-Markov-Modellen (HMM) aufgebaut sein.

Das erfindungsgemäße System bietet eine Reihe von wesentlichen Vorteilen gegenüber dem Stand der Technik.

Zum einen ermöglicht es ein computergestütztes kognitives Training auch bei Patienten, die aufgrund des Grades ihrer Behinderung bisher davon ausgeschlossen waren. Darüber hinaus ergibt sich eine Erweiterung des Trainingsangebotes auf motorische Übungen mit großen Freiheitsgraden hinsichtlich Bewegungsarten und beobachtbarem Volumen, wobei vor allem auch ein unmittelbares Feedback sowohl an den Patienten über die Güte seiner motorischen Übungen mittels automatischer Klassifikation möglich ist, wobei die Einteilung in Güteklassen durch positive und negative Beispiele beim "Teach-in" festgelegt werden kann als auch eine entsprechende Rückmeldung an den Therapeuten stattfinden kann.

Schließlich ermöglicht das erfindungsgemäße System eine Erweiterung des computergestützten Trainings um neue Komponenten, die nur mit Gestikerkennung möglich sind, wie z. B. spezielles Bewegungstraining und Gestikspiele, wie beispielsweise Knobeln. So kann beispielsweise der Computer mit dem Patienten das beliebte Spiel Papier/Schere/Stein spielen, bei dem die flach ausgestreckte Hand Papier, die gespreizten Mittelund Zeigefinger Schere und die geballte Faust Stein bedeuten. Dies lässt sich natürlich für einen behinderten Patienten abwandeln, indem der Computer in der patientenspezifischen Adaptionsphase andere Gesten für die entsprechenden Begriffe erlernt und dann später auch akzeptiert.

Weitere Vorteile, Merkmale und Einzelheiten der Erfindung ergeben sich aus der nachfolgenden Beschreibung eines Ausführungsbeispiels sowie anhand der Zeichnung. Dabei zeigen:
- Fig. 1: eine Diagrammdarstellung des Adaptionsprozesses, bei dem den Symbolen oder Befehlen entsprechende Gesten des Patienten zugeordnet werden,
- Fig. 2: den jeweils vor dem Einsatz eines Trainingsprogramms mit einem Gestikerkennungssystem durchzuführenden Check zur Überprüfung der aktuellen Gestik des Patienten mit den abgespeicherten Daten, und
- Fig. 3: ein Ablaufdiagramm eines computergestützten Rehabilitationstrainings mit einer ein solches Gestikerkennungssystem umfassenden Eingabevorrichtung.

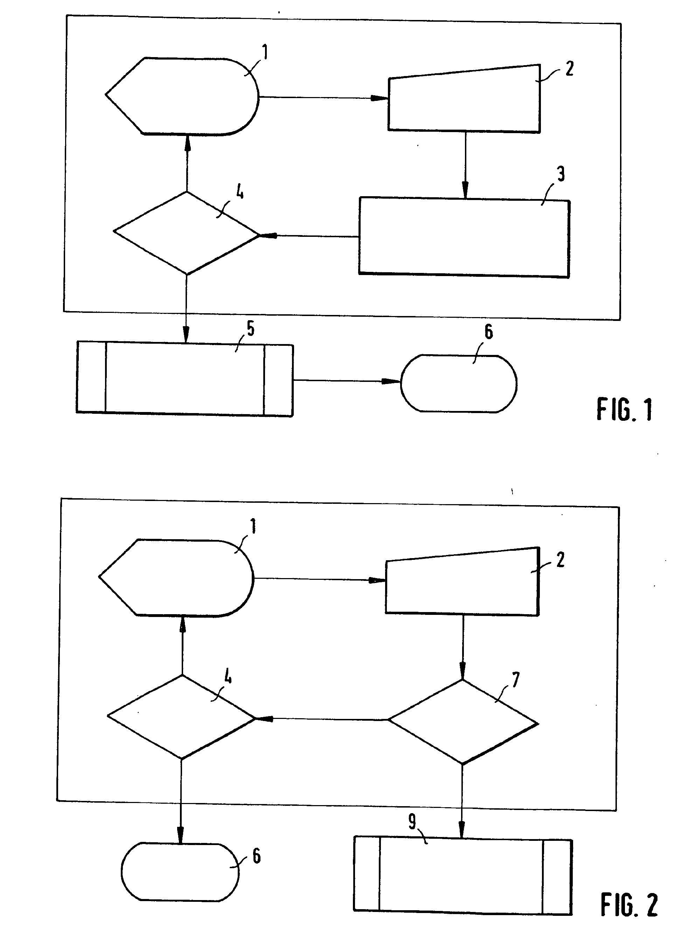

Für alle in Frage kommenden Symbole, dabei umfasst der Begriff Symbole auch alle möglichen Eingabebefehle wie beispielsweise Halt, Vorwärts, Zurück, Links, Rechts oder dergleichen, wird in einer in Fig. 1 gezeigten Adaptionsphase in Verbindung mit dem Therapeuten jeweils ein Symbol i auf dem Bildschirm gezeigt und nach dieser Präsentation des Symbols die Reaktion des Patienten, also dessen daraufhin erfolgende Gestik oder Handbewegung oder dergleichen, festgehalten. Der Stufe 1 des Präsentierens des Symbols i folgt also die Stufe 2 der Reaktionserkennung und als Stufe 3 eine Festlegung, ob die entsprechende Reaktionscharakteristik geeignet ist, um über ein Gestikerkennungsprogramm dem Computer die Möglichkeit zu geben aus dieser Reaktion, also aus dieser speziellen Geste zu erkennen, dass das gerade gezeigte Symbol i aus einer Reihe von Symbolen 1 - m vom Patienten gemeint ist. In der Stufe 4 wird festgehalten, ob alle Symbole auf diese Art und Weise durch Reaktionen des Patienten abgedeckt sind. Ist dies nicht der Fall, so wird das nächste Symbol i+1 in der Stufe 1 wiederum aufgerufen. Sind alle Symbole erkannt und ihre zugeordnete Gestik akzeptiert worden, so wird das Adaptionsmuster in der Stufe 5 gespeichert, so dass dann als Stufe 6 das eigentliche Rehabilitationstraining gestartet werden kann.

Beim späteren Checkprogramm vor jedem Training zur Rehabilitationsunterstützung mit Hilfe eines Computers erfolgt wiederum für alle Symbole in der Stufe 1 eine Präsentation der einzelnen Symbole nacheinander, woraufhin in Stufe 2 wiederum die Reaktion des Patienten überprüft und in einer Stufe 7 festgestellt wird, ob die Reaktion mit der gespeicherten Reaktion des Adaptionsmusters in Stufe 5 in Fig. 1 soweit übereinstimmt, dass das System ihr das Symbol i zugewiesen hat. Ist dies der Fall, wird in Stufe 4 überprüft, ob alle Symbole erkannt worden sind, falls nicht wird zur Stufe 1 zurückgeschaltet und das nächste Symbol aufgerufen. Wenn aber alle Symbole erkannt worden sind, so wird das Trainingsprogramm gestartet (Stufe 6). Wenn dagegen in der Erkennungsstufe 7 die Reaktion des Patienten auf die Präsentation des Symbols i nicht dem abgespeicherten Muster entspricht, so erfolgt ein Wiedereinstieg in das Adaptionsverfahren nach Fig. 1 was in Fig. 2 als Stufe 9 dargestellt ist.

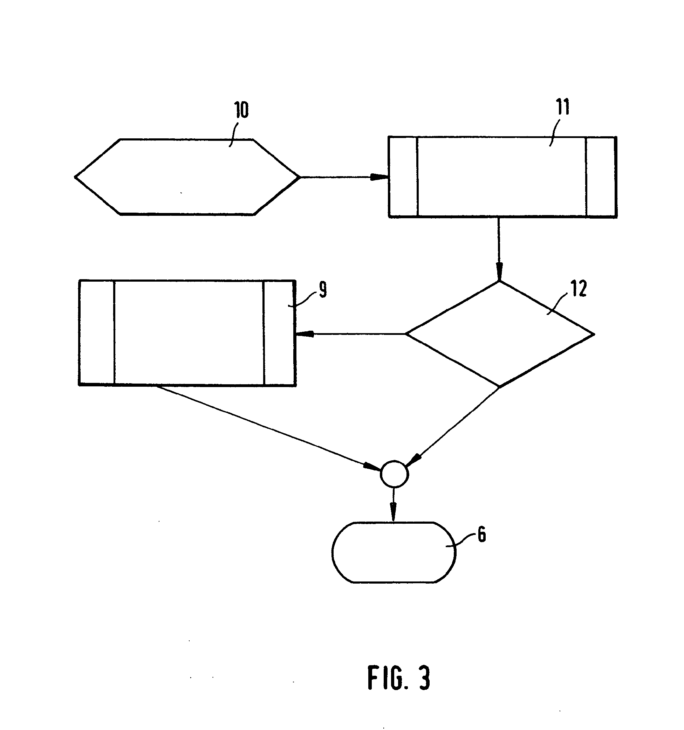

Unter Verwendung dieses Adaptionsverfahrens und des Gestikchecks gemäß den Fig. 1 und 2 ist schematisch eine Trainingssitzung unter Verwendung der Gestiksteuerung in Fig. 3 gezeigt. Nach dem Starten des Systems in Stufe 10 erfolgt der Gestikcheck gemäß Fig. 2, in Fig. 3 als Stufe 11 dargestellt. Ergibt dieser in Stufe 12, dass eine neue Adaptionsphase notwendig ist, so wird auf das Adaptionsverfahren nach Fig. 1 weitergeschaltet (Stufe 9) in Fig. 3. Bedarf es aber einer Neuadaption nicht, so wird direkt das Trainingsprogramm 6 gestartet wie dies auch der Fall ist, nachdem in Stufe 13 der Adaptionsprozess erneut durchgeführt wurde.

## Patentansprüche

1. Vorrichtung zur interaktiven Rehabilitationsunterstützung durch computergestütztes, selbständig vom Patienten durchgeführtes Training mit einer Eingabevorrichtung für die Eingabebefehle des Patienten, **dadurch gekennzeichnet, dass** die Eingabevorrichtung ein Gestikerkennungssystem als zusätzliches Eingabemedium umfasst.

2. Vorrichtung nach Anspruch 1, **dadurch gekennzeichnet, dass** das Gestikerkennungssystem in wiederholten Adaptionsphasen patientenspezifisch programmierbar ist.

3. Vorrichtung nach Anspruch 2, **dadurch gekennzeichnet, dass** das Gestikerkennungssystem auf der Grundlage von Hidden-Markov-Modellen (HMM) aufgebaut ist.

4. Vorrichtung nach einem der Ansprüche 1 bis 3, **dadurch gekennzeichnet, dass** das Gestikerkennungssystem vor dem Einsatz als Eingabevorrichtung für das Trainingsprogramm alle gespeicherten Symbole abspielt und die Gestik des Benutzers mit den gespeicherten Gestikdaten vergleicht.

5. Vorrichtung nach einem der Ansprüche 1 bis 4, **dadurch gekennzeichnet, dass** die betreuende Stelle (Klinik oder Therapeut) auf die gespeicherten Gestikdaten zugreifen kann.

6. Vorrichtung nach einem der Ansprüche 1 bis 5, **dadurch gekennzeichnet, dass** das Trainingsprogramm Bewegungstraining und Gestikspiele umfasst.

7. Vorrichtung nach einem der Ansprüche 1 bis 6, **dadurch gekennzeichnet, dass** es ein System zur Gütebewertung der motorischen Übungen mittels automatischer Klassifikation umfasst.

8. Vorrichtung nach Anspruch 7, **dadurch gekennzeichnet, dass** die Güteklasseneinteilung im Zuge des "Teach-in" während der Adaptionsphasen festlegbar ist.
